# EUROPEAN PATENT APPLICATION

(11) **EP 4 043 442 A1**
(43) Date of publication of application: **17.08.2022**
(21) Application number: 21156802.7
(22) Date of filing: 12.02.2021
(51) Int. Cl.: C07D 239/54, A61K 31/513, A61P 25/28

(54) **6-METHYLURACIL DERIVATIVES WITH ANTICHOLINESTERASE ACTIVITY AND THEIR USE**

(71) Applicant: AVVA Pharmaceuticals Ltd, 4001 Mesa Geitonia, Limassol (CY)
(72) Inventor: PETROV, Konstantin Aleksandrovich, 420100 Kazan (RU); SEMENOV, Vyacheslav Engelsovich, 420132 Kazan (RU); ZUEVA, Irina Vladimirovna, 420136 Kazan (RU); MUKHAMEDYAROV, Marat Aleksandrovich, 420070 Kazan (RU); SINYASHIN, Oleg Geroldovich, 421001 Kazan (RU)
(74) Representative: D Young & Co LLP

(57) **Abstract**

The present invention relates to compounds of the Formula (I): 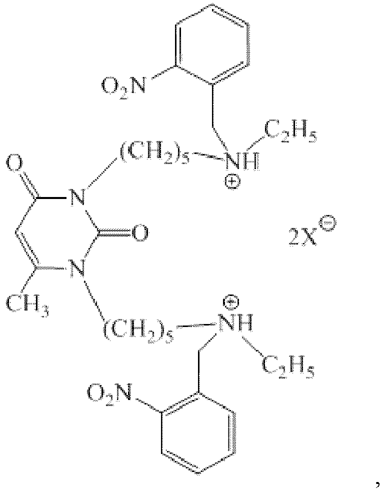 in which X represents Cl or Br. According to the present invention, the compounds have anticholinesterase activity. The present invention also relates to pharmaceutical compositions containing the compounds of the present invention, and the application of the compounds of the present invention for the treatment of Alzheimer's disease.

## Description

### Background of the invention

The invention relates to new derivatives of 6-methyluracil with anticholinesterase activity, pharmaceutical compositions containing them, and their use in the treatment of Alzheimer's disease.

Alzheimer's disease is the most common neurodegenerative disease characterized by an increase in the production of pathological amyloid beta. The main risk factor for the disease is an age: after 60 years, the incidence of the disease doubles every five years and reaches 85% by the age of 85. The disease is accompanied by dementia, multiple cognitive deficits, advancing memory impairments: patients show multiple disturbances of cognitive function, including memory disorder associated with a poor acquisition of new information and reproduction of previously acquired knowledge.

Central cholinergic deficiency is considered to play a key role in the pathogenesis of Alzheimer's disease, it underlies cognitive disorders and determines the severity of the clinical manifestations of the disease.

Cholinesterase inhibitors are used clinically for the pharmacological correction of the consequences of defects underlying Alzheimer's disease and myasthenia gravis. Their effectiveness is also shown in the treatment of glaucoma, intestinal atony, traumatic brain injuries, alcoholism, manic-depressive psychoses, schizophrenia, senile dementia, autism, sleep disorders, etc. The pharmaceutical industry produces many anticholinesterase drugs, such as aminostigmine, deoxypeganine, stefaglabrin, distigmine, quinolitin, oxazil, pyridostigmine, proserin, galantamine, physostigmine, and donepezil. Depending on the chemical structure and physical and chemical properties, anticholinesterase drugs differ among themselves, for example, tertiary amines (physostigmine, galantamine, donepezil, etc.) penetrate biological membranes, including the blood-brain barrier, and have a pronounced effect on the central nervous system. Since the mid-1990s, acetylcholinesterase (AChE) inhibitors have been used in the treatment of patients with mild or moderate Alzheimer's disease as cognitive enhancers. However, the application of common AChE inhibitors is effective only in relation to the main symptoms of this disease (memory impairment), but, unfortunately, does not influence the pathogenesis of the disease. This disadvantage significantly limits the application of these drugs. There are prerequisites for believing that in the case of Alzheimer's disease, AChE inhibitors are also able to influence the pathogenesis of the disease, reducing the aggregation of amyloid beta. However, this statement is true only for those AChE inhibitors, binding sites of which include the so-called "peripheral anionic site" of this enzyme. Unfortunately, the number of AChE inhibitors that bind to the "peripheral anionic site" of AChE is extremely small.

In the search for effective AChE inhibitors, a new class of cholinesterase inhibitors was proposed - alkylammonium derivatives of 6-methyluracil, highly selective blockers of mammalian acetylcholinesterase [RAS reports. - 1998. -Vol. 362, No. 1. P. 68-70: [RAS reports. - 2001. - Vol. 376, No. 6. - P. 818-822; Modern toxicol. problems. - 2004. - No. 3. - P. 25-33; RAS reports. - 2005. - Vol. 401, No. 1. - P. 120-123; Chem. Pharm. J. - 2005. - Vol. 39, No. 5. - P. 15-19; Modern Toxicol. Issues. - 2006. - No. 2. - P. 13-22; Chem. Biol. Interactions. - 2008. - Vol.175. - P. 286-292]. Some of the alkylammonium derivatives of 6-methyluracil have been proposed for the treatment of pathological muscle weakness syndromes. Besides that, their binding sites for the enzyme were determined by computational methods — in the region of the "peripheral anionic site" of AChE [MedChemComm. - 2014. -- Vol. 5. - No. 11. - P. 1729-1735]. A library of compounds, for which peripheral anionic site is one of the binding sites with AChE as indicated by molecular docking, was created. These compounds were synthesized, examined for their inhibitory activity on human AChE *in vitro,* and their acute toxicity was evaluated in mice [ChemMedChem. - 2015 -Vol. 10. No. 11. - P.1863-1874.].

In the RU patent No. 2565756, 1,3-bis[5-(*ortho*-nitrobenzyl-ethylamino)pentyl-1]-6-methyluracil is claimed for the treatment of Alzheimer's disease, and its dose for restoring memory characteristics in transgenic mice of B6C3-Tg(APP695)85Dbo Tg(PSENI)85Dbo line has been established. In accordance with the described experiments, the number of amyloid deposits was analyzed in the brain of experimental mice. It was shown that for daily intraperitoneal use, this compound in the dose of 5 mg/kg is sufficient to reduce the number of amyloid deposits in the cerebral cortex of transgenic mice on average by 50%.

However, the free base 1,3-bis[5-(*ortho*-nitrobenzyl-ethylamino)pentyl-1]-6-methyluracil has several limitations in its use. For instance, it is an oily substance, insoluble in water, so that the pharmaceutical industry is not interested in it. In addition, it is quite toxic (LD₅₀ 51.48 mg/kg, in mice, i.p.).

Therefore, the need for low-toxic and highly effective acetylcholinesterase inhibitors remains urgent. Furthermore, the pharmaceutical industry needs water-soluble acetylcholinesterase inhibitors to facilitate the production of drugs.

### Detailed Disclosure of the Invention

The objective of this invention is to satisfy the above-mentioned needs, in particular in providing new water-soluble acetylcholinesterase inhibitors for the treatment of Alzheimer's disease, which have lower toxicity and higher activity compared to known acetylcholinesterase inhibitors. In addition, the objective of the invention is to extend the range of known means for this purpose.

The inventors of the invention have developed new derivatives of 6-methyluracil, which, as it was unexpectedly discovered, are highly selective inhibitors of the enzyme acetylcholinesterase and have significantly lower toxicity and significantly higher anticholinesterase activity compared to known anticholinesterase agents, and are also soluble in water.

Therefore, this invention relates to compounds of the Formula (I) 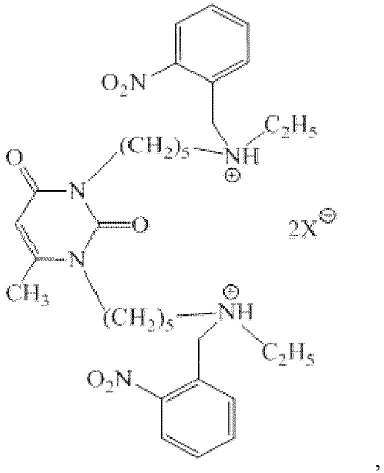 wherein X represents Cl or Br.

The compounds of Formula (I) are dihydrohalides of 1,3-bis[5-(*ortho*-nitrobenzyl-ethylamino)pentyl-1]-6-methyluracil and have anticholinesterase activity.

The invention also relates to a compound of the Formula (I) for use in the treatment of Alzheimer's disease.

The invention also relates to the use of a compound of the Formula (I) in the manufacture of a medicament for the treatment of Alzheimer's disease.

The invention also relates to a method of treatment of Alzheimer's disease in a patient in need thereof, comprising administering to the patient an effective amount of a compound of the Formula (I).

The invention also relates to pharmaceutical compositions comprising a therapeutically effective amount of the compound of the Formula (I) and one or more pharmaceutically acceptable excipients.

The term "therapeutically effective amount" used in this claim means the amount of compounds of the Formula (I), which, in combination with its activity and toxicity indicators, as well as on the basis of specialist knowledge, is effective in this pharmaceutical composition and ensure the achievement of this therapeutic effect. Besides, the therapeutically effective amount depends on body weight, route of administration, individual response to the active substance, type of the product, and time or interval during which the application is performed.

The pharmaceutically acceptable excipient may be any known pharmaceutically acceptable excipient, in particular, it may be a carrier or diluent; it is usually mixed with the active compound, or diluted, or incorporated into the active compound. As to a diluent, the carrier may be a solid, semi-solid, or liquid material, acting as an excipient or carrier for the active compound. Compositions may also include wetting agents, emulsifiers, preservatives, humectants, and/or flavours. Examples of all of these are well known to those skilled in the pharmaceutical arts.

### Examples

The present invention is described by the following non-limiting Examples below.

### Production Examples

### General scheme for the production of compounds of the Formula (I)

Compounds of the Formula (I) according to the present invention was prepared by alkylation of 1,3-bis(5-ethylaminopentyl-1)-6-methyluracil (3) by *ortho*-nitrobenzylbromide (4) followed by hydrohalogenation *in situ* with concentrated hydrobromic acid (5) or concentrated hydrochloric acid (6) according to the synthesis scheme below. The compound (3) and the method for its preparation are described [Russian Chemical Bulletin - 2003. - Vol. 52. -No. 7. - P.1511-1515],

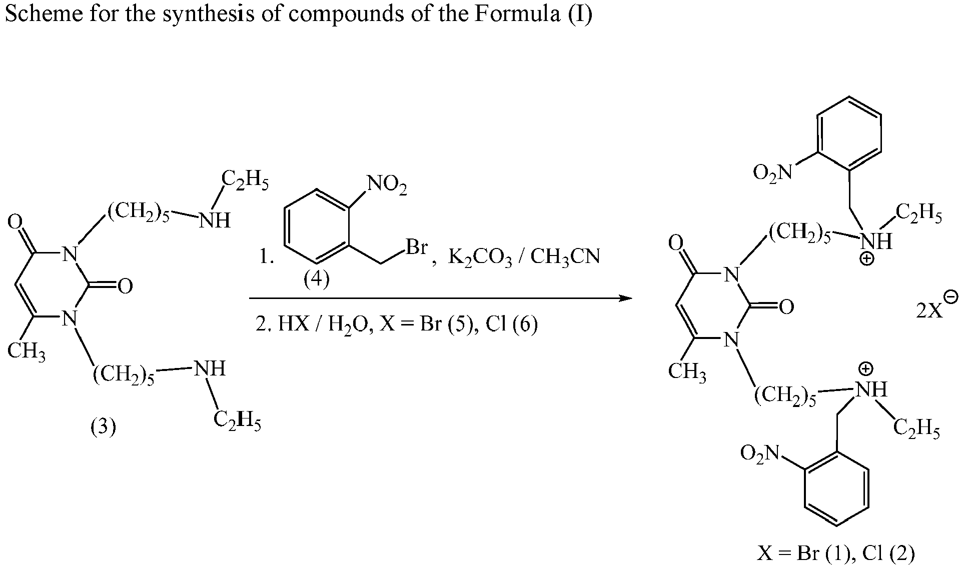

### Example 1. Preparation of 1,3-bis[5-(ortho-nitrobenzylethylamino)pentyl]-6-methyluracil dihydrobromide (1)

Add 5.18 g (37.5 mmol) of potassium carbonate and 5.4 g (25.0 mmol) of ortho-nitrobenzyl bromide (4) to a solution of 4.4 g (12.5 mmol) of the compound (3) in 100 ml of acetonitrile, stir the reaction mass at a temperature of 65-70°C for 10 h, following which filter off, evaporate the filtrate in vacuum, and add 100 ml of distilled water and 5 ml (62.0 mmol) of a 48% aqueous solution of HBr (5) to the residue. Stir the reaction mass at room temperature for 24 hours and then filter. Heat the filtrate to 50°C, add 2 g of activated carbon, and filter off after cooling. Evaporate the filtrate to dryness in vacuum, add 150 ml of methyl alcohol and 5 g of calcined magnesium sulfate. After 10 hours, filter off the desiccant, evaporate the filtrate in vacuum and add 50 ml of diethyl ether to the residue. Triturate the residue in ether, decant. Repeat the procedure two more times. Dry the residue to give 8.30 g (85%) of compound (1) as a pale orange powder.

**1,3-Bis[5-(*ortho*-nitrobenzylethylamino)pentyl]-6-methyluracil dihydrobromide** (1) has the following physical and chemical characteristics: m.p. 95-101 °C; NMR ¹H (500 MHz) in CDCl₃, δ mass fraction: 1.24-1.27 (m, 6H, 2NCH₂CH₃), 1.47-1.50 (m, 4H, 2CH₂), 1.61-1.68 (m 4H, 2CH2), 1.92-1.96 (m, 4H, 2CH2), 2.26 (s, 3H, Cᵤᵣ CH3), 3.12-3.32 (m, 8H, 4CH2), 3.83-3.87 (t, 2H, NᵤᵣCH2, *J*= 7.6 Hz), 3.89-3.92 (t, 2H, NᵤᵣCH₂, *J*= 6.5 Hz), 4.64-4.70 (m, 4H, 2NC*H*₂Ph), 5.60 (s, 1H, CᵤᵣH), 7.65 (m, 2H, 2ArH), 7.81 (m, 2H, 2ArH) 8.06 (m, 2H, 2ArH), 8.66 (m, 2H, 2ArH), 11.35 (br. s, 2H, 2N⁺H); ¹H NMR spectrum (600 MHz) in D₂O, δm.f: 1.37-1.45 (m, 10H, 2NCH₂CH₃, 2CH₂), 1.61-1.70 (m, 4H, 2CH₂), 1.81-1.90 (m, 4H, 2CH₂), 2.31 (s, 3H, CᵤᵣCH₃), 3.27-3.38 (m, 8H, 4CH2), 3.83-3.87 (m, 4H, 2NᵤᵣCH₂), 4.65-4.70 (m, 4H, 2NCH2 Ph), 5.73 (s, 1H, CᵤᵣH), 7.75 (m, 4H, 4ArH), 7.87 (m, 2H, 2ArH), 8.31 (m, 2H, 2ArH); ¹³C NMR spectrum (400 MHz) in CDCl₃, δm.f: 8.9, 15.1, 20.0, 22.5, 22.8, 23.7, 26.7, 28.1, 39.7, 39.9, 40.2, 44.6, 48.2, 48.4, 52.3, 53.3, 65.5, 101.3, 124.5, 124.6, 125.8, 131.5, 134.6, 135.1, 135.2, 148.8, 148.9, 151.8, 162.0. IR spectrum in a thin layer, v cm⁻¹: 2944 (s), 2865 (s), 2631 (s), 1694 (s), 1653 (s.p.), 1532 (s.p.), 1470 (s), 1432 (s), 1344 (s), 1205 (w), 1108 (w), 866 (w), 795 (w).; MALDI-MS (matrix *-para-* nitroaniline): 621.4 [MH-2HBr] ⁺, 622.4 [M-2HBr] ⁺ , calculated for C₃₃H₄₈Br₂N₆O₆ 621.3, 622.4, respectively. Found, %: C 50.57, H 6.13, Br 20.43, N 10.68. Molecular formula C₃₃H₄₈Br₂N₆O₆. Calculated, %: C 50.52, H 6.17, Br 20.37, N 10.71.

The resulting compound is a hygroscopic powder with a colour from light orange to orange, soluble in chloroform, dimethylformamide, dimethyl sulfoxide, ethyl alcohol, and water.

### Example 2. Preparation of 1,3-bis[5-(ortho-nitrobenzylethylamino)pentyl]-6-methyluracil dihydrochloride (2)

Add 5.18 g (37.5 mmol) of potassium carbonate and 5.4 g (25.0 mmol) of *ortho*-nitrobenzyl bromide (4) to a solution of 4.4 g (12.5 mmol) of the compound (3) in 100 ml of acetonitrile, stir the reaction mass at a temperature of 65-70°C for 10 h, following which filter off, evaporate the filtrate in vacuum, and add 100 ml of distilled water and 6 ml (60.0 mmol) of a 36% aqueous solution of HCl (6) to the residue. Stir the reaction mass at room temperature for 24 hours and then filter. Heat the filtrate to 50°C, add 2 g of activated carbon, and filter off after cooling. Evaporate the filtrate to dryness in vacuum, add 150 ml of methyl alcohol and 5 g of calcined magnesium sulfate. After 10 hours, filter off the desiccant, evaporate the filtrate in vacuum and add 50 ml of diethyl ether to the residue. Triturate the residue in ether, decant. Repeat the procedure two more times. Dry the residue to obtain 71.30 g (82%) of the compound (2) as a yellowish powder.

**1,3-Bis[5-(*ortho*-nitrobenzylethylamino)pentyl]-6-methyluracil dihydrochloride (2)** has the following physicochemical characteristics: m.p. 64-67 °C; ¹H NMR spectrum (600 MHz) in D₂O, δ m.f.: 1.36-1.50 (m, 10H, 2NCH₂CH₃, 2CH₂), 1.63 (m, 2H, CH₂), 1.69 (m, 2H, CH2), 1.80 (m, 2H, CH2), 1.88 (m, 2H, CH2), 2.31 (s, 3H, CᵤᵣCH₃), 3.24-3.27 (m, 4H, 2NCH2), 3.29-3.33 (m, 4H, 2NCH₂), 3.83-3.87 (m, 4H, 2NᵤᵣCH₂), 4.57-4.65 (m, 4H, 2NC*H*₂Ph), 5.76 (s, 1H, CᵤᵣH), 7.70 (m, 2H, 2ArH), 7.75 (m, 2H, 2ArH), 7.86 (m, 2H, 2ArH), 8.31 (m, 2H, 2ArH); ¹H NMR (500 MHz) in DMSO-d₆, δm.f.: 1.30-1.33 (m, 10H, 2NCH₂CH₃, 2CH2), 1.55 (m, 4H, 2CH2), 1.81 (m, 4H, 2CH2), 2.30 (s, 3H, CᵤᵣCH₃), 3.07 (m, 4H, 2NCH₂), 3.16 (m, 4H, 2NCH₂), 3.73-3.77 (m, 4H, 2N_{yp}CH₂), 4.59-4.64 (m, 4H, 2NC*H*₂Ph), 5.65 (s, 1H, CᵤᵣH), 7.76 (m, 2H, 2ArH), 7.84 (m, 2H, 2ArH), 8.12 (m, 2H, 2ArH), 8.20 (m, 2H, 2ArH), 10.61 (1H, NH), 10.75 (1H, NH); NMR spectrum ¹³C (400 MHz) D₂O, δ, m.f.: 7.9, 14.2, 19.2, 22.5, 22.6, 23.1, 23.2, 26.3, 27.3, 41.1, 45.2, 48.5, 52.5, 54.5, 66.0, 100.9, 124.7, 126.5, 132.2, 135.3, 135.5, 148.4, 152.8, 155.5, 164.6. IR spectrum in a thin layer, v cm⁻¹ : 2430 (os), 2948 (s), 2865 (sl), 2634 (sl), 1695 (s), 1652 (os), 1532 (os), 1471 (s), 1432 (s), 1344 (s), 1204 (sl), 1108 (sl), 866 (sl), 795 (sl); MALDI-MS (matrix *- para-*nitroaniline): 621.4 [MH-2HCl]⁺, 622.4 [M-2HCl]⁺, calculated for C₃₃H₄₈Cl₂N₆O₆621.3, 622.4, respectively. Found, %: C 56.97, H 6.95, Cl 10.19, N 12.08. Molecular formula C₃₃H₄₈Br₂N₆O₆. Calculated, %: C 57.04, H 7.00, Cl 10.17, N 12.02.

The resulting compound is a light yellow hygroscopic powder, soluble in dimethylformamide, dimethyl sulfoxide, ethyl alcohol, and water.

### Biological Activity

### Example 3. Determination of the anticholinesterase activity of the compounds of the Formula (I) according to the present invention

Registration of the enzymatic reaction was carried out according to the Ellman method [Ellman G.L., Courtney K.D., Andres V. Jr., Feather-Stone R.M. A new and rapid colorimetric determination of acetylcholinesterase activity. Biochem Pharmacol 1961, N°7, C.88-95]. The analysis of the dependence of cholinesterase activity on the concentration of the inhibitor was carried out by the Hill equation with the calculation of the average effective concentration - IC₅₀. Human acetylcholinesterase and human butyrylcholinesterase were purchased from Sigma-Aldrich.

**Table 1. Anticholinesterase activity and toxicity of the compounds (1) and (2)**

| | AChE of human red blood cells, ICso mol/l | BChE of human serum, IC₅₀, mol/l | LD₅₀, mg/kg (in mice, i.p.) | LD₅₀, mg/kg (in mice, p.o.) |
|---|---|---|---|---|
| Compound (1) | 3.2×10⁻¹⁰ | 3.0×10⁻⁵ | 199 | 520 |
| Compound (2) | 1.2×10⁻¹⁰ | 2.3×10⁻⁵ | 150 | 290 |
| 1,3-bis[5-(ortho-nitrobenzyl-ethylamino)pentyl-1]-6-methyluracil free base | 7.1×10⁻⁹ | 3.0×10⁻⁷ | 51 | - |
| Donepezil hydrochloride | 6.7×10⁻⁹ | 7.9×10⁻⁶ | 5 | 45 |

| | | | | |
|---|---|---|---|---|
| Compound (1) - 1,3-bis[5-(*ortho*-nitrobenzylethylamino)pentyl]-6-methyluracil dihydrobromide Compound (2) - 1,3-bis[5-(*ortho*-nitrobenzylethylamino)pentyl]-6-methyluracil dihydrochloride | | | | |

The results of the inhibitory activity of the compounds of the Formula (I) according to the present invention, shown in Table 1, indicate that:
- they are highly selective AChE inhibitors (efficacy against acetylcholinesterase is by five orders of magnitude higher than against butyrylcholinesterase);
- anticholinesterase activity is an order of magnitude higher than that of 1,3-bis[5-(ortho-nitrobenzyl-ethylamino)pentyl-l]-6-methyluracil free base and donepezil hydrochloride.

### Example 4. Determination of the general toxic effect of the compounds of the Formula (I) according to the present invention

The study of the general toxic effect of the claimed compounds was carried out according to the "Guidelines for preclinical studies of drugs." [M: Grif and K, 2012. Ed. A.N. Mironova] on mice of the CD-1 line ("Puschino" Nursery, Moscow Region), both males and females, weighing 19.0 ± 2.0 g, kept on a standard diet in the day/night lighting regimen (12 hours) at 22 °C.

The summary of the toxicological evaluation of the compounds are shown in Table 1. For comparison, the LD₅₀ of 1,3-bis[5-(*ortho*-nitrobenzyl-ethylamino)pentyl-1]-6-methyluracil (free base compound) is 51 mg/kg on intraperitoneal administration, i.e. the compound (1) of the Formula (I) according to the present invention is almost 4 times less toxic, and the compound (2) of the Formula (I) according to the present invention is almost 3 times less toxic. As compared to donepezil hydrochloride (a drug traditionally used in the treatment of Alzheimer's disease), intraperitoneal administration of the compound (1) of the Formula (I) is 40 times less toxic, and administration of the compound (2) of the Formula (I) is 30 times less toxic according to the present invention, and when administered orally, according to the invention, the compounds (1) and (2) of the Formula (I) are 12 and 6 times less toxic, respectively.

### Example 5. Treatment of Alzheimer's disease symptoms using the compound (1) in the genetic model in mice

The compound (1) of the Formula (I) according to the present invention was tested *in vivo* for the ability to improve the working memory of mice in the conditions of the genetic model of Alzheimer's disease. The tests were performed on transgenic mice with genotype B6C3-Tg(APP695)85Dbo Tg(PSENI)85Dbo expressing the chimeric mouse/human amyloid precursor protein and mutant human presenelin-1. The line was purchased at The Jackson Laboratory (USA) and kept in the "Pushchino" nursery for laboratory animals (Pushchino, Moscow Region). This line of transgenic mice is characterized by an increase in the number of amyloid-beta deposits (plaques) in the brain with age. At the beginning of the research, the age of the transgenic mice was 9 months.

The conclusions about the spatial memory were made based on the testing mice with food pre-deprivation (feed reduction to 2.5 grams per day) in a T-maze in the "rewarding alternation" model for 14 days [Robert M J Deacon, J Nicholas P Rawlins. T-maze alternation in the rodent. Nature protocols. 2006. - Vol. 1. - N 1. P. 7-12]. The criterion of training was satisfied when animals performed more than 80% of correct entries into the sleeve with food for 3 consecutive days.

Twenty minutes before the start of the experiment, the experimental groups of transgenic animals (n = 9-10) were orally administered the compound (1) of the Formula (I) from a 0.01% and 0.1% aqueous solution at doses of 1 mg/kg and 10 mg/kg, respectively. The comparison treatment group was orally administered donepezil hydrochloride from a 0.01% aqueous solution (Sigma-Aldrich) at a dose of 1 mg/kg. An equivalent amount of water was orally administered to control groups of transgenic and non-transgenic mice.
The results are shown in Tables 2 and 3 below.

**Table 2. The average percentage of choice of the right direction by mice in the "rewarded alternation" test in a T-maze when using the compound (1) of Formula (I)**

| Group of animals | The average percentage of choosing the right direction | p-value * |
|---|---|---|
| Water (Tg-) | 68±3 | |
| Water (Tg+) | 59 ±2* | 0.017 |
| Compound (1), 1 mg/kg | 62 ±3 | 0.145 |
| Compound (1), 10 mg/kg | 65±2 | 0.597 |
| Donepezil hydrochloride, 1 mg/kg | 66±2 | 0.583 |
| *Note: * - the difference with the control group (Tg-) is statistically significant at p≤0.05. (Comparison with Mann-Whitney test) | | |

**Table 3. The percentage of trained mice in the "rewarding alternation" test in the T-maze when using the compound (1) of Formula (I)**

| Group of animals (number of animals in the group) | The percentage of trained mice (%) | p-value * |
|---|---|---|
| Water (Tg-), (n = 7) | 71 | |
| Water (Tg+), (n = 9) | 11* | 0.035 |
| Compound (1), 1 mg/kg, (n = 10) | 40 | 0.03348 |
| Compound (1), 10 mg/kg, (n = 9) | 56 | 0.6329 |
| Donepezil hydrochloride, 1 mg/kg, (n = 10) | 50 | 0.6221 |
| *Note: * - the difference with the control group (Tg-) is statistically significant at p≤0.05. (comparison was performed using Fisher's exact test) | | |

As far as after applying the compound (1) of the Formula (I) according to the present invention in doses of 1 mg/kg and 10 mg/kg, there were no statistically significant differences in the percentage of choosing the right direction in the T-maze compared with healthy animals (Tables 2 and 3), the compound (1) of the Formula (I) according to the present invention, when administered orally, is effective for correction of memory impairment.

### Example 6. Treatment of Alzheimer's disease symptoms using the compound (2) in the conditions of the genetic model in mice

The compound (1) of the Formula (I) according to the present invention was tested *in vivo* for the ability to improve the working memory of mice in the conditions of the genetic model of Alzheimer's disease. The tests were performed on transgenic mice with genotype B6C3-Tg(APP695)85Dbo Tg(PSENI)85Dbo expressing the chimeric mouse/human amyloid precursor protein and mutant human presenelin-1. The line was purchased at The Jackson Laboratory (USA) and kept in the "Pushchino" nursery for laboratory animals (Pushchino, Moscow Region) . This line of transgenic mice is characterized by an increase in the number of amyloid-beta deposits (plaques) in the brain with age. At the beginning of the research, the age of the transgenic mice was 8 months.

The conclusions about the spatial memory were made based on the testing mice with food pre-deprivation (feed reduction to 2.5 grams per day) in a T-maze in the "rewarding alternation" model for 14 days [Robert M J Deacon, J Nicholas P Rawlins. T-maze alternation in the rodent. Nature protocols. 2006. - Vol. 1. - N 1. P. 7-12]. The criterion of training was satisfied when animals performed more than 80% of correct entries into the sleeve with food for 3 consecutive days.

20 minutes before the start of the experiment, the experimental groups of transgenic animals (n = 9-10) were orally administered the compound (2) of the Formula (I) with a 0.01%, 0.05% and 0.1% aqueous solution in doses of 1 mg/kg, 5 mg/kg and 10 mg/kg, respectively. The comparison drug group was orally administered donepezil hydrochloride from a 0.05% aqueous solution (Sigma-Aldrich) at a dose of 5 mg/kg. An equivalent amount of water was orally administered to control groups of transgenic and non-transgenic mice. The results are shown in Tables 4 and 5.

**Table 4. The average percentage of the choice of the right direction by mice in the "rewarding alternation" test in a T-maze when using the compound (2) of Formula (I)**

| Group of animals | The average percentage of choosing the right direction | p-value * |
|---|---|---|
| Water (Tg-) | 77 ±3 | |
| Water (Tg+) | 43 ±3*** | 0.000 |
| Compound (2), 1 mg/kg | 72 ±2 | 0.185 |
| Compound (2), 5 mg/kg | 75 ±3 | 0.650 |
| Compound (2), 10 mg/kg | 69±3 | 0.068 |
| Donepezil hydrochloride, 5 mg/kg | 69±2 | 0.054 |
| *Note: *** - the difference with the control group (Tg-) is statistically significant at p≤0.001. (Comparison with Mann-Whitney test) | | |

**Table 5. The percentage of trained mice in the "rewarding alternation" test in the T-maze when using the compound (1) of the Formula (I)**

| Group of animals (number of animals in the group) | The percentage of trained mice | p-value * |
|---|---|---|
| Water (Tg-), (n = 10) | 100 | |
| Water (Tg+), (n = 10) | 0*** | 0.0003 |
| Compound (2), 1 mg/kg, (n = 10) | 60 | 0.0867 |
| Compound (2), 5 mg/kg, (n = 10) | 100 | 1.000 |
| Compound (2), 10 mg/kg, (n = 9) | 67 | 0.0867 |
| Donepezil hydrochloride, 5 mg/kg, (n = 10) | 70 | 0.1544 |
| *Note: *** - the difference with the control group (Tg-) is statistically significant at p≤0.001. (comparison is performed using Fisher's exact test) | | |

As far as after applying the compound (2) of the Formula (I) according to the present invention in doses of 1 mg/kg, 5 mg/kg and 10 mg/kg, there were no statistically significant differences in the percentage of choosing the right direction in the T-maze compared with healthy animals (Tables 4 and 5), the compound (2) of the Formula (I) according to the present invention, when administered orally, is effective for correcting memory impairment.

### Example 7. Testing the compound (1) for the ability to reduce the number of amyloid plaques in the cortex of animals with a genetic model of Alzheimer's disease

The compound (1) of the Formula (I) according to the present invention was tested *in vivo* for the ability to reduce the number of amyloid plaques in the brain of transgenic mice of the B6C3-Tg(APP695) 85Dbo Tg(PSENI)85Dbo genotype expressing the chimeric mouse/human amyloid precursor protein and mutant human presenelin-1. The line was purchased at The Jackson Laboratory (USA) and kept in the "Pushchino" nursery for laboratory animals Pushchino, Moscow Region). At the beginning of the research, the age of the transgenic mice was 9 months.

Experimental groups of transgenic animals (n = 9-10) were orally administered the compound (1) of the Formula (I) at doses of 1 mg/kg and 10 mg/kg, respectively. The reference group was orally administered donepezil hydrochloride at a dose of 1 mg/kg. An equivalent amount of water (Water (Tg+)) was orally administered to control groups of transgenic mice.

Amyloid-beta deposits were evaluated after sacrifice on mouse brain sections (20 µm) using the Thioflavin S fluorescent dye and the Olympus BX51W1 fluorescence microscope, by the method described in the article [http://library.med.utah.edu/WebPath/histhtml/manuals/manuals.html]. The results are shown in Table 6.

**Table 6. The number of amyloid plaques in the brain of transgenic mice (per HPF, 10x magnification), which were administered compound (1) of the Formula (I)**

| Group of animals | Number of amyloid plaques in the cortex | p-value * |
|---|---|---|
| Water (Tg+) | 40±2 | |
| Compound (1), 1 mg/kg | 28±2*** | 0.000 |
| Compound (1), 10 mg/kg | 22±1*** | 0.000 |
| Donepezil hydrochloride, 1 mg/kg | 45±2 | 0.054 |
| *Note: *** - the difference with the control group (Tg-) is statistically significant at p≤0.001. (comparison was performed using Mann-Whitney test) | | |

The staining results of the prefrontal cortex on aggregated amyloid beta, shown in Table 6, indicate a significant decrease in the number of amyloid-beta plaques. The administration of donepezil hydrochloride did not significantly affect the number of amyloid plaques in the cortex. This fact allows us to consider the compound (1) of the formula (I) according to the present invention as more effective product for prevention of the formation of amyloid plaque than donepezil hydrochloride.

### Example 8. Testing the compound (2) for the ability to reduce the number of amyloid plaques in the cortex of animals with a genetic model of Alzheimer's disease

Compound (2) of the Formula (I) according to the present invention was tested *in vivo* for the ability to reduce the number of amyloid plaques in the brain of transgenic mice of the B6C3-Tg(APP695)85Dbo Tg(PSENI)85Dbo genotype expressing the chimeric mouse/human amyloid precursor protein and mutant human presenelin-1. The line was purchased at The Jackson Laboratory (USA) and kept in the "Pushchino" nursery for laboratory animals Pushchino, Moscow Region). At the beginning of the research, the age of the transgenic mice was 8 months.

Experimental groups of transgenic animals (n = 9-10) were orally administered the compound (2) of the Formula (I) at doses of 1 mg/kg, 5 mg/kg, and 10 mg/kg, respectively. The reference group was orally administered donepezil hydrochloride at a dose of 5 mg/kg. An equivalent amount of water (Water (Tg+)) was orally administered to control groups of transgenic mice.

Amyloid-beta deposits were evaluated after sacrifice on mouse brain sections (20 µm) using the Thioflavin S fluorescent dye and the Olympus BX51W1 fluorescence microscope, by the method described in the article [http://library.med.utah.edu/WebPath/histhtml/manuals/manuals.html]. The results are shown in Table 7.

**Table 7. The number of amyloid plaques in the brain of transgenic mice (per HPF, 10x magnification), which were administered compound (2) of the Formula (I)**

| Group of animals | Number of amyloid plaques in the cortex | p-value * |
|---|---|---|
| Water (Tg+) | 12± 1 | |
| Compound (2), 1 mg/kg | 11±1 | 0.143 |
| Compound (2), 5 mg/kg | 7±0,5* | 0.023 |
| Compound (2), 10 mg/kg | 7±0,4*** | 0.000 |
| Donepezil hydrochloride monohydrate, 5 mg/kg | 12± 1 | 0.055 |
| *Note: * - difference with the control group (Tg+) is statistically significant at p≤0.05, *** - at p≤0.001 (comparison was performed using Mann-Whitney test) | | |

The staining results of the prefrontal cortex on aggregated amyloid beta, shown in Table 7, indicate a significant decrease in the number of amyloid beta plaques. The administration of donepezil hydrochloride did not significantly affect the number of amyloid plaques in the cortex. This fact allows us to consider that the compound (2) of the Formula (I) according to the present invention is more effective than donepezil hydrochloride for preventing the formation of amyloid plaques.

### Results Summary - Advantages of the Invention

The compounds of formula (I) of the present invention are 1,3-bis[5-(*ortho-*nitrobenzyl-ethylamino)pentyl-1]-6-methyluracil dihydrohalides. The above results show that the compounds have the following properties:
- highly selective inhibitors of the acetylcholinesterase enzyme, and their anticholinesterase activity is an order of magnitude higher than that of the free base 1,3-bis[5-(*ortho*-nitrobenzyl-ethylamino)pentyl-1]-6-methyluracil and donepezil hydrochloride;
- stop the development of Alzheimer's disease symptoms and more effectively reduce the number of amyloid plaques in the brain of transgenic mice compared with donepezil hydrochloride in a similar dose;
- have good solubility in water;
- have low toxicity for anticholinesterase agents compared with both the free base 1,3-bis[5-(*ortho*-nitrobenzyl-ethylamino)pentyl-1]-6-methyluracil and with donepezil hydrochloride.

### Example 9. Production of a pharmaceutical composition

For obtaining the pharmaceutical composition, the compound of the present invention was mixed as an active ingredient in a pharmaceutically effective amount with a pharmaceutically acceptable carrier (e.g., water) according to pharmaceutical methods well known to those skilled in the art.

## Claims

1. A compound of the Formula (I): 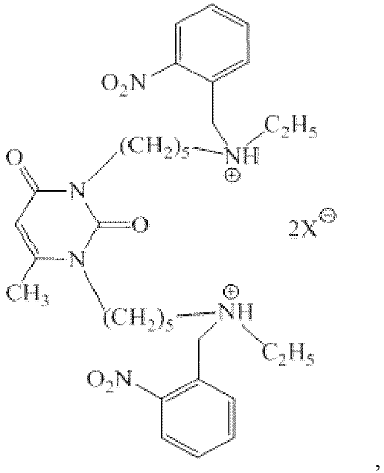 wherein X represents Cl or Br.

2. A compound of the Formula (I) according to claim 1 for use in the treatment of Alzheimer's disease.

3. Use of a compound of the Formula (I) according to claim 1 in the manufacture of a medicament for the treatment of Alzheimer's disease.

4. A method of treatment of Alzheimer's disease in a patient in need thereof, comprising administering to the patient an effective amount of a compound of the Formula (I) according to claim 1.

5. A pharmaceutical composition comprising a therapeutically effective amount of a compound of the Formula (I) according to claim 1 and one or more pharmaceutically acceptable excipients.
